(19)

Europäisches
Patentamt
European
Patent Office
Office européen
des brevets

(11) **EP 2 659 839 A1**

(12) **EUROPEAN PATENT APPLICATION**
published in accordance with Art. 153(4) EPC

(43) Date of publication:
**06.11.2013 Bulletin 2013/45**

(51) Int Cl.:
***A61B 8/00*** *(2006.01)*

(21) Application number: **11852262.2**

(22) Date of filing: **07.12.2011**

(86) International application number:
**PCT/JP2011/078233**

(87) International publication number:
**WO 2012/090658 (05.07.2012 Gazette 2012/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.12.2010 JP 2010290652**

(71) Applicant: **Hitachi Medical Corporation Chiyoda-ku Tokyo 101-0021 (JP)**

(72) Inventor: **FUJII, Nobuhiko Tokyo 101-0021 (JP)**

(74) Representative: **Strehl Schübel-Hopf & Partner Maximilianstrasse 54 80538 München (DE)**

(54) **ULTRASONIC DIAGNOSIS DEVICE AND IMAGE PROCESSING METHOD**

(57) Provided is an ultrasonic diagnostic apparatus comprising an STIC function, wherein, in order to make it possible to reproduce images with the correct time phase by preventing erroneous image reproduction due to time phase estimation failure, the ultrasonic diagnostic apparatus comprises: a time phase estimation unit that uses echo data to estimate time phase information of periodic movement of an examination object included in the echo data; an image processing unit that uses the echo data and the time phase information estimated by the time phase estimation unit to create an ultrasonic image of the examination object at each time phase; and, in addition, a time phase correction unit that corrects the time phase information estimated by the time phase estimation unit. The image processing unit creates an ultrasonic image using the time phase information corrected by the time phase correction unit.

FIG.3

START

301 — ACQUISITION OF 2-DIMENSIONAL ECHO DATA

302 — CONVERSION INTO IMAGE DATA, STORAGE IN MEMORY

303 — TIME PHASE ESTIMATION CALCULATING PROCESS

304 — NEED CORRECTION? No / Yes

309 — TIME PHASE CORRECTION CALCULATING PROCESS

305 — VOLUME DATA CONSTRUCTION PROCESS

306 — VOLUME RENDERING PROCESS

307 — IMAGE DISPLAY

308 — SET CORRECTION? No / Yes

END

EP 2 659 839 A1

**Description**

Field of the Invention

The present invention relates to an ultrasonic diagnostic apparatus, in particular to an image processing technique that processes 3-dimensional image data obtained by the ultrasonic diagnostic apparatus.

Description of Related Art

An ultrasonic diagnostic apparatus is capable of collecting 3-dimensional echo data by mechanically or electronically scanning ultrasonic beams in the direction orthogonal to the ultrasonic beams, for creating and displaying a 3-dimensional image from 3-dimensional echo data.

However, acquisition of 3-dimensional echo data requires a certain period of time, thus a target object such as the heart of a fetus which beats at high rate fluctuates during data acquisition which makes it difficult to obtain 3-dimenisonal images that are appropriate for diagnosis.

Considering the above-mentioned problem, a technique is proposed that estimates the time phase of periodic movement of a target, then extracts and images the data of each time phase from the 3-dimensional echo data (Patent Document 1). This technique is referred to as STIC (Spatial Temporal Image Correlation), which enables the moving-image display by consecutively displaying images of the respective time phases. The STIC collects the echo data while moving the scan positions at low speed, and extracts the time variation of the luminance in the collected echo data. By calculating auto-correlation function or performing fast Fourier transform with respect to the change curve which is acquired as above, the frequency of variation is acquired for estimating the time phase of the 3-dimensional echo data.

Prior Art Documents

Patent Documents

Patent Document 1: JP-A-2005-74225

Summary of Invention

Technical Problem

However, since the STIC estimates the time phase from the echo data, in a case such as when the information amount on a target included in the echo data is low or when the movement of the object other than the target region is dominant, there is a possibility that the estimation may fail. In that case, the images are reproduced with incorrect cycles and the target cannot be displayed correctly. In such cases, the echo data must be measured and corrected again, which causes excessive burdens both on examiners and examinees especially when the images are to be reproduced at a place which is temporally and locally apart from the examination.

The objective of the present invention is to provide an ultrasonic diagnostic apparatus capable of preventing regeneration of incorrect images due to time phase estimation failure and regenerating correct time phase images without imposing a burden on an examiner and examinee, whereby enhancing the effect of the STIC technology.

Brief Summary of the Invention

The present invention solves the above-described problem by adding a function to correct the time phase which is estimated by an ultrasonic diagnostic apparatus provided with a time-phase estimation function.

That is, the ultrasonic diagnostic apparatus of the present invention comprises:

an ultrasonic probe configured to transmit ultrasonic beams and receive the reflected echo signals from an examination object;
a beam forming unit configured to supply driving signals to the ultrasonic probe;
a signal processing unit configured to receive the reflected echo signals, process the signals, and generate the echo data;
a time phase estimation unit configured to estimate the time phase information of the periodic movement in the examination object included in echo data using the echo data;
an image processing unit configured to create an ultrasonic image of the examination object for each time phase using the time phase information estimated by the time phase estimation unit and the echo data;

a display unit configured to display the processing result of the image processing unit; and

a time phase correction unit configured to correct the time phase information estimated by the time phase estimation unit,

wherein the image processing unit creates the ultrasonic image using the time phase information corrected by the time phase correction unit.

In addition, time phase information in the present invention includes, in addition to the time phase itself, the frequency (heart rate for a heart) and the cycles for inducing the time phase.

Effect of the Invention

In accordance with the present invention, a unit configured to correct the time phase estimated by a time phase estimation unit is comprised, thus images can be reproduced with correct time phase without re-acquiring the echo data which enables regeneration of images with correct time phase without re-acquisition of the echo data, providing 3-dimensional ultrasonic images with high accuracy.

Brief Description of the Drawings

Fig. 1 is a block diagram showing the general configuration of the ultrasonic diagnostic apparatus to which the present invention is applied.

Fig. 2 is a block diagram showing a configuration example of a control unit in the ultrasonic diagnostic apparatus related to the present invention.

Fig. 3 is a flowchart showing the operation procedure of the ultrasonic diagnostic apparatus by Embodiment 1.

Figs. 4(A)~(D) are views for explaining a time phase estimation process of a time phase estimation unit in Embodiment 1.

Figs. 5(A)-(C) are views for explaining the process of a volume data construction processing unit.

Figs. 6(A)~(C) are examples of a display screen of a display unit.

Fig. 7 is another example of the display screen of the display unit.

Fig. 8 is a flowchart showing the operation procedure of the ultrasonic diagnostic apparatus by Embodiment 2.

Figs. 9(A) and (B) are views for explaining a time phase estimation process of a time phase estimation unit in Embodiment 3.

Detailed Description of the Invention

Embodiments of the present invention will be described below.

<Embodiment 1>

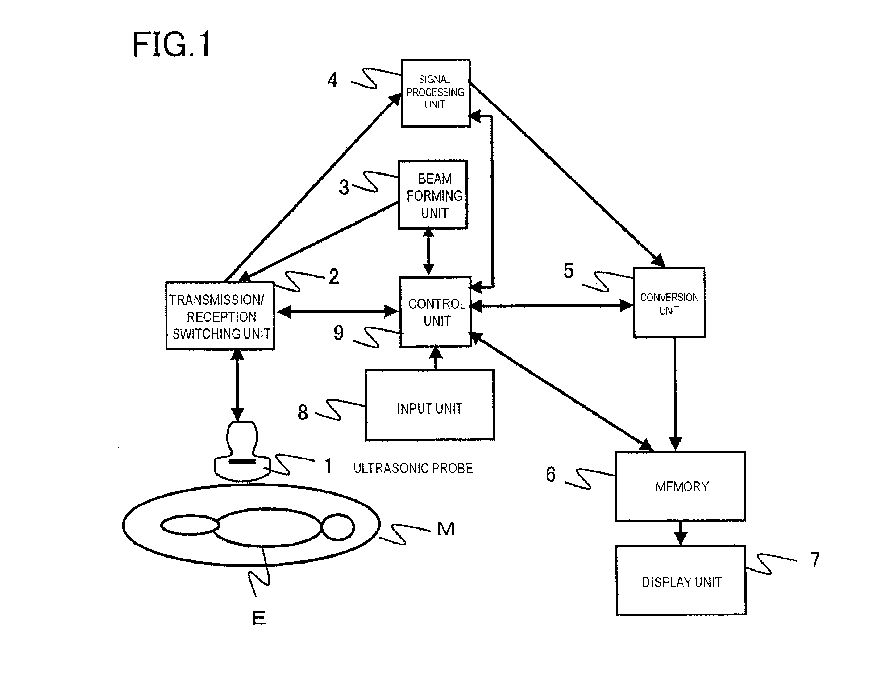

Fig. 1 shows the general configuration of the ultrasonic diagnostic apparatus to which the present invention is applied. The ultrasonic diagnostic apparatus comprises an ultrasonic probe 1, a transmission/reception switching unit 2, a beam forming unit 3, a signal processing unit 4, a conversion unit 5, a memory 6, a display unit 7, an input unit 8 and a control unit 9.

The ultrasonic probe 1 transmits ultrasonic beams to an imaging object and receives the reflected echo signals from the imaging object. Here, fetus E in the uterus of mother M will be exemplified as an imaging object. The configuration of the ultrasonic probe 1 or the kind of transducers that form the probe is not limited to any particular type.

For example, the ultrasonic probe 1 can be either one of a 1-dimensional ultrasonic probe in which plural channels of transducer elements are arrayed in the major-axis direction of the ultrasonic probe or a 2-dimensional ultrasonic probe in which plural channels of transducer elements are arrayed also in the minor-axis direction in addition to the major-axis direction. Also, the transducers that form the ultrasonic probe 1 can be either one of those using piezo elements or those using a semiconductor referred to as CMUT (Capacitive Michromachined Ultrasonic Transducer: IEEE Trans. Ultrason. Ferroelect. Freq. Contr. Vol. 45 pp. 678-690 May 1998, etc.).

The transmission/reception switching unit 2 switches the functions of transmission and the reception in the ultrasonic probe 1. The transmission/reception switching unit 2 supplies the transmission signals to the ultrasonic probe 1 from the beam forming unit 3 when the ultrasonic probe 1 functions as a transmitter, and also receives the reflected echo signals from the object (mother M) and outputs the signals to the signal processing unit 4 when the ultrasonic probe functions as a transmitter.

The beam forming unit 3 forms the signals for the ultrasonic probe 1 to transit ultrasonic beams to the object. Also, the beam forming unit 3 is capable of focusing beams in the transmission or reception also in the minor-axis direction by changing the delay times to be given to the respective transducer elements in the minor-axis direction of the ultrasonic probe 1. Further, the beam forming unit 3 is configured to perform weighting on the ultrasonic transmission signals by changing the amplitude of the ultrasonic transmission signals to be given to the respective transducer elements in the minor-axis direction and perform weighting on the ultrasonic reception signals by changing the amplification degree or attenuance of the ultrasonic reception signals from the respective transducer elements in the minor-axis direction. The beam forming unit 3 is also capable of performing aperture control by driving the respective transducer elements in the minor-axis direction.

The signal processing unit 4 makes the input reflected echo signals into echo data by amplifying and digitalizing the signals.

The conversion unit 5 converts the echo data into 2-dimensional ultrasonic image data.

The memory 6 stores 2-dimensional ultrasonic image data, 3-dimensional ultrasonic image data, and the parameters, etc. that are necessary for the operation of the apparatus.

The display unit 7 displays the ultrasonic image, etc. that are stored in the memory 6, and is formed by a device such as a CRT monitor or liquid-crystal monitor. The output method of the display unit 7 can be either one of the analogue output or digital output, which is capable of displaying ultrasonic images for an operator to make diagnosis. The display unit 7 can also function as a GUI along with the input unit 8 to be described later, for displaying the processing result, etc. of the control unit 9.

The input unit 8 is for inputting control parameters of the ultrasonic diagnostic apparatus such as the parameters for imaging the ultrasonic image data, and is configured by at least one device such as a keyboard, a trackball or a mouse.

The control unit 9 controls the respective functions of the transmission/reception switching unit 2, beam forming unit 3, signal processing unit 4, conversion unit 5, memory 6 and display unit 7 on the basis of the parameters input by the input unit 8. Also, the control unit 9 is configured by a computer system centering on a central processing unit, for performing various calculations and so on necessary for image processing.

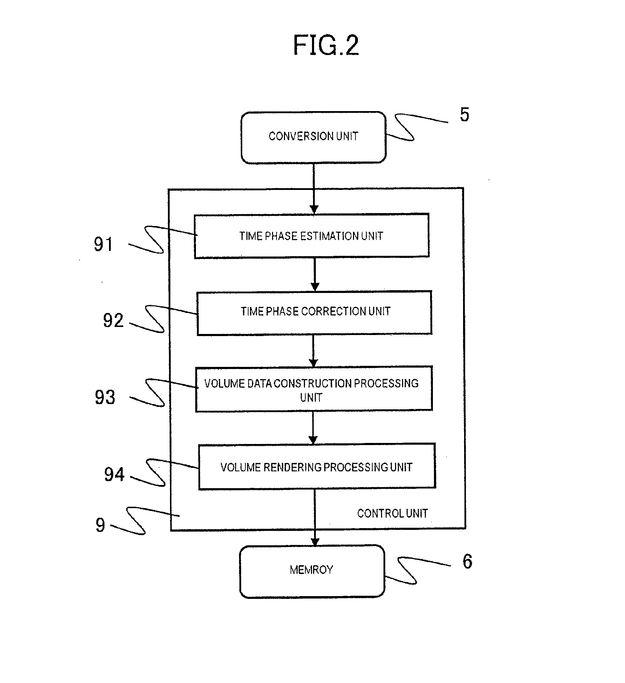

Fig. 2 is a configuration example of the control unit 9 for actualizing the functions for image processing calculation. As shown in the diagram, the control unit 9 comprises a time phase estimation unit 91, a time phase correction unit 92, a volume data construction processing unit 93 and a volume rendering processing unit 94.

The time phase estimation unit 91 calculates the cycle (time phase) of the movement of an imaging object, using the 2-dimensional ultrasonic image converted in the conversion unit 5. The processing result of the time phase estimation unit 91 is displayed on the display unit 7. The image processing unit creates a 3-dimensional ultrasonic image of the examination object for each time phase using the time phase information estimated by the time phase estimation unit 91 and the echo data.

The time phase correction unit 92 corrects the movement cycles or the time phases of the imaging object calculated by the time phase estimation unit 91 using the correction value set by a user via the input unit 8. Or, the time phase correction unit 92 automatically corrects the movement cycles or time phases of the imaging object using the processing result in the time phase estimation unit 91.

The volume data construction processing unit 93 which is a part of the image processing unit, using the time phase estimated by the time phase estimation unit 91 or the time phase corrected by the time phase correction unit 92, selects the ultrasonic image data at the same time phase from among the plural sets of ultrasonic image data of the imaging object obtained by 3-dimensional imaging and creates 3-dimensional image data (volume data).

The volume rendering processing unit 94 which is a part of the image processing unit performs processing such as a projection process of MIP, MinIP, etc. or a process of cutting out a desired cross-section on the volume data, and creates an image such as a volume rendering image.

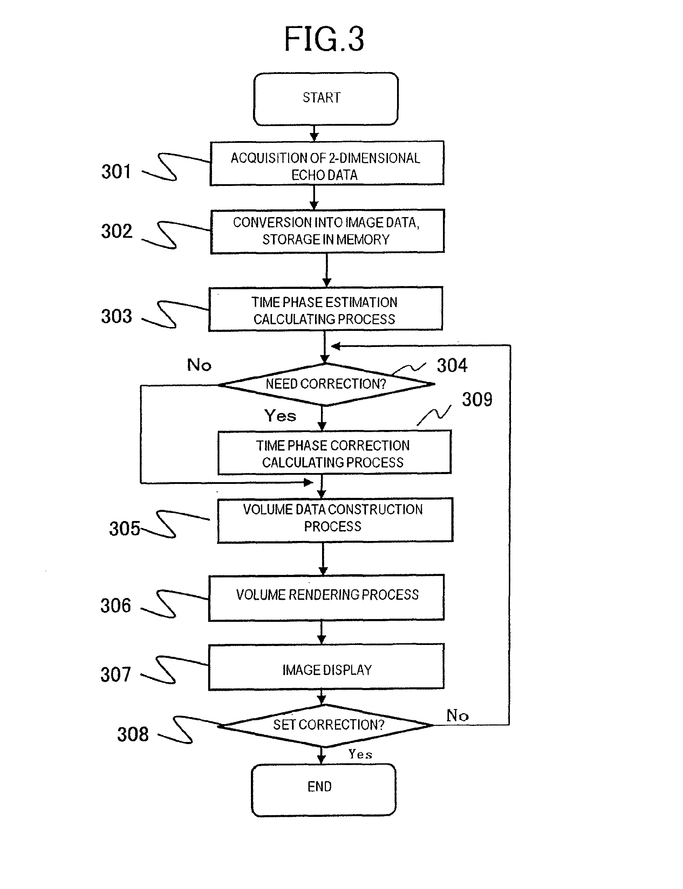

Next, the operation of the above-described ultrasonic diagnostic apparatus will be described. The procedure of the operation is indicated in Fig. 3. The imaging operation in the ultrasonic diagnostic apparatus in the present embodiment is the same as the conventional ultrasonic diagnostic apparatus up to the acquisition of 2-dimensional image data. Simply put, the probe 1 is applied to an abdominal region of an imaging object who is a pregnant woman here, transmission of ultrasonic beams and reception of the reflected echo signals are performed, and the 2-dimensional echo data along the beam cross-section is acquired. The acquisition of 2-dimensional echo data is repeated while moving the ultrasonic beam in the direction orthogonal to the beam cross-section with comparatively low speed, and plural sets of 2-dimensional echo data are obtained (step 301). Information such as the width of the ultrasonic beam, the focusing position, etc. is set by the input parameters via the input unit 8 so that an observation target which is the heart of a fetus here can be depicted. Other information such as the speed for moving the ultrasonic beam or the moving range is set in the same manner.

Such acquired plural sets of 2-dimensional echo data are respectively converted into 2-dimensional image data by the conversion unit 5 and stored in the memory 6 (step 302). The plural sets of 2-dimensional image data are acquired at different times and cross-sectional positions, and they potentially have the time phase information of the movement in a case that the imaging object executes periodic movement.

Thus the time phase estimation unit 91 calculates the movement cycle of the imaging object from the 2-dimensional image data, and estimates the time phase at the time that the respective sets of 2-dimensional echo data are acquired (step 303).

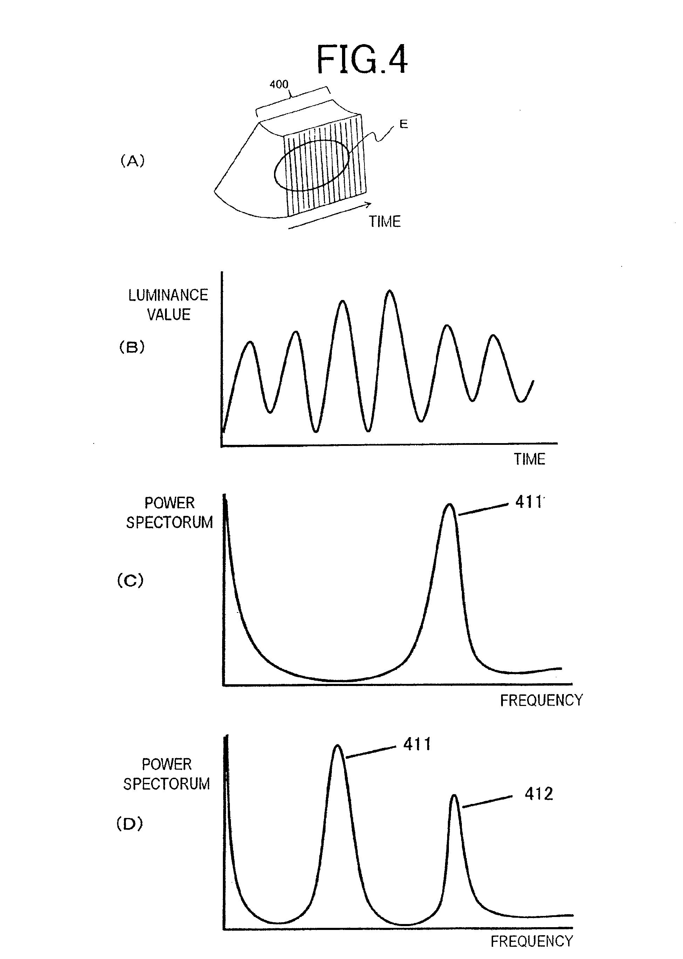

An example of the time phase estimation method to be executed by the time phase estimation unit 91 will be described referring to Fig. 4. Fig. 4(A) shows plural 2-dimensional ultrasonic images obtained by imaging, for example the heart of a fetus. With respect to each of the plural 2-dimensional ultrasonic images, for example the average of the image luminance is calculated. The image luminance keeps changing along with the change of fetal blood volume caused by repeated cardiac constriction and dilation. This change is represented by a waveform of the luminance average as shown in Fig. 4(B). In the diagram, the lateral axis indicates the acquisition time or acquired time of the plural 2-dimensional ultrasonic images. The time phase estimation unit 91 acquires the frequency spectrum by executing Fourier transform on the time change of the average luminance. An example of the frequency spectrum is indicated in Fig. 4(C) and Fig. 4(D).

In a case that the movement of an imaging object includes only constant-frequency movement, the frequency spectrum is indicated as a single peak 411 shown in Fig. 4(C). Though it is ideal to acquire a single peak for estimating the time phase, two or more peaks 411, 412 as shown in Fig. 4(D) are often generated in reality since noise vibration such as the movement of a mother is included.

The time phase estimation unit 91 estimates, in the frequency spectrum, the frequency of the peak when a single peak is acquired and the frequency having the greatest power spectrum when there are two or more peaks, as the movement frequency of the imaging object, then calculates the entire time phase of the cardiac movement of the fetus from the acquired movement frequencies. The processing result of the time phase estimation unit 91 is displayed on the display unit 7 along with a rendering image to be described later.

Next, the volume data construction processing unit 93, when there is no need for correcting the time phase estimated by the time phase estimation unit 91 or no command is issued (step 304), selects plural sets of 2-dimensional image data of the same time phase from the plural 2-dimensional image data stored in the memory 6 using the time phase information of the imaging object calculated in the time phase estimation unit 91, and constructs a 3-dimensional ultrasonic image (step 305).

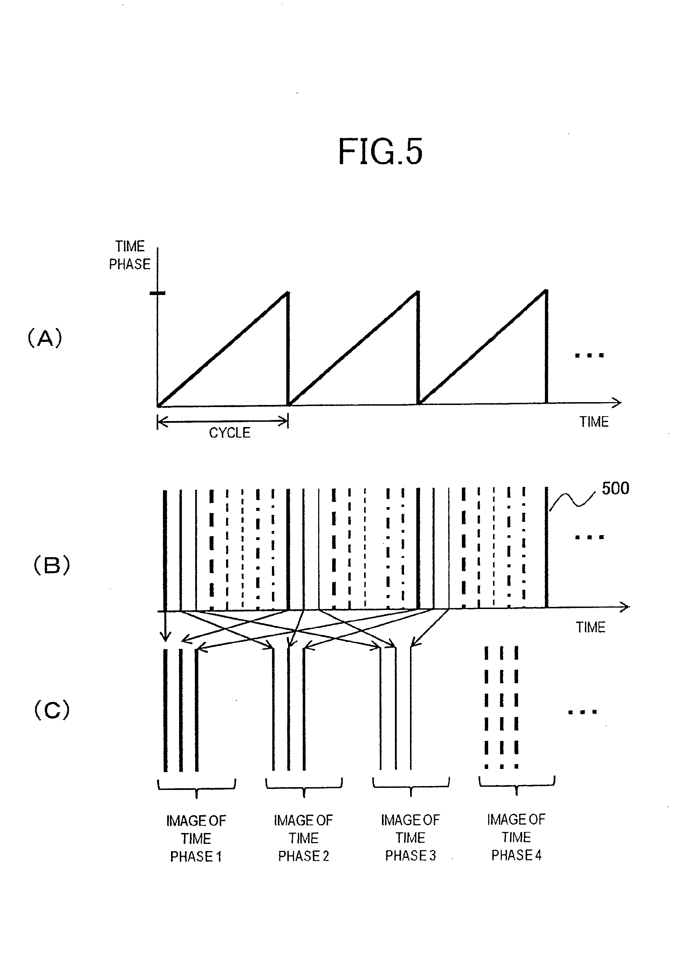

Fig. 5 is the explanatory view of the above-described processing. Fig. 5 (A) is the time phase of the movement cycle which is estimated in the time phase estimation unit 91. Fig. 5 (B) shows image data 500 which is plural sets of 2-dimensional echo data acquired at the same time axis as that of (A) or created from the acquired 2-dimensional echo data, corresponding to image data 400 in Fig. 4(A). In Fig. 5(B), for an explanatory reason, plural sets of image data are represented by different line types for each time phase, and image data sets at the same time phase are represented by the same line type. The volume data construction processing unit 93 creates 3-dimensional image data (volume data) for every time phase as shown in Fig. 5(C) using the time phase information in Fig. 5(A).

The volume rendering processing unit 94 processes the volume data constructed by the volume data construction processing unit 93 using the commonly known method for depicting 3-dimensional ultrasonic images (rendering method), and creates images to be displayed on the display unit 7 (step 306). For example, in the volume rendering method, ray casting is performed on the pixel values of a 2-dimensional ultrasonic image distributed in a 3-dimensional space while providing opacity, and a translucent image is ultimately generated by adding the opacity values. The volume rendering method is capable of depicting a target by enhancing it in almost opaque condition, by setting a large value of opacity on the voxels corresponding to the cardiac surface of the fetus to be displayed.

Also, another shading method may be used such as the surface rendering method using a shading model or the depth method.

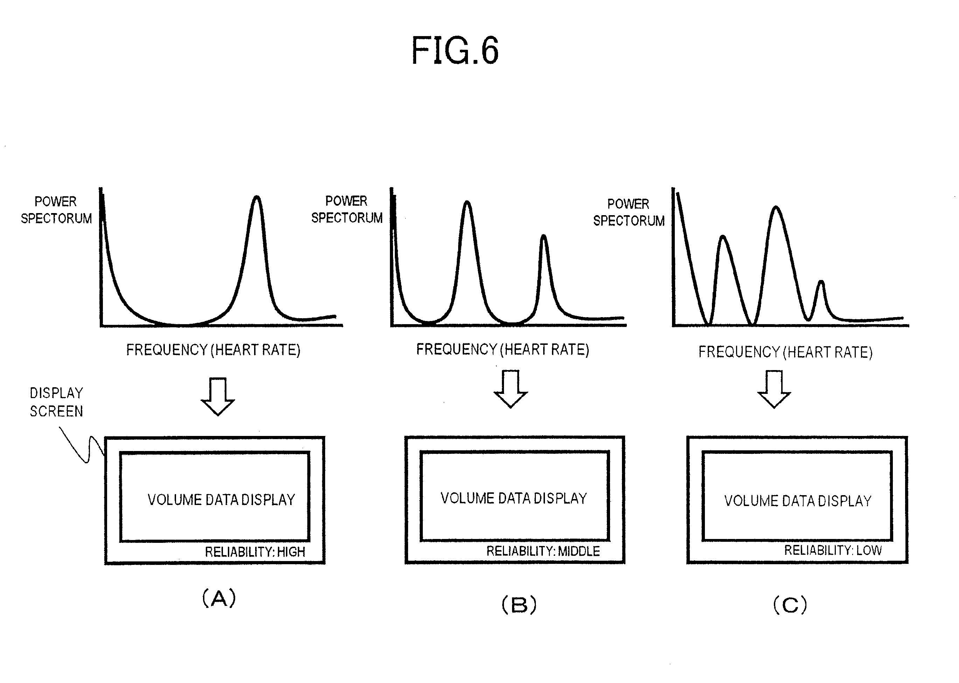

The images created in the volume rendering processing unit 94 are displayed on the display unit 7 along with the processing result in the previously-described time phase estimation unit 91 (step 307). Fig. 6 shows an example of the display. Fig. 6(A) is a case that there is a single peak in the frequency spectrum acquired in the time phase estimation unit 91, and the indication of "reliability: high" is displayed with the image since the reliability of the time phase estimated in the time phase estimation unit 91 is assumed to be high. Fig. 6(B) is a case that there are two peaks and the frequency of the peak having a larger power spectrum is estimated as the movement frequency, of which the reliability is lower than that of (A). That is, there is a possibility that the frequency having a smaller spectrum may be the movement frequency of the imaging object. In such a case, an indication such as "reliability: middle" is displayed. In a case that there are more than three peaks, the reliability of the estimated time phase is even lower, thus an indication such as "reliability: low" is displayed as shown in (C).

The display method of reliability is not limited to the example shown in Fig. 6. It may be displayed graphically, the frequency spectrum indicated in the upper part of Fig. 6 may be displayed, or the numeric value of the selected frequency may be displayed on the frequency spectrum. In that case, the numeric values may be calculated in terms of, for example the heart rate instead of the frequency may be displayed for the lateral axis of the frequency spectrum. By displaying the heart rate, a user can determine the reliability of the time phase estimated by the time phase estimation unit 91 from the selected frequency (heart rate) and the proximity or remoteness of the heart rate of a fetus that he/she knows from experience.

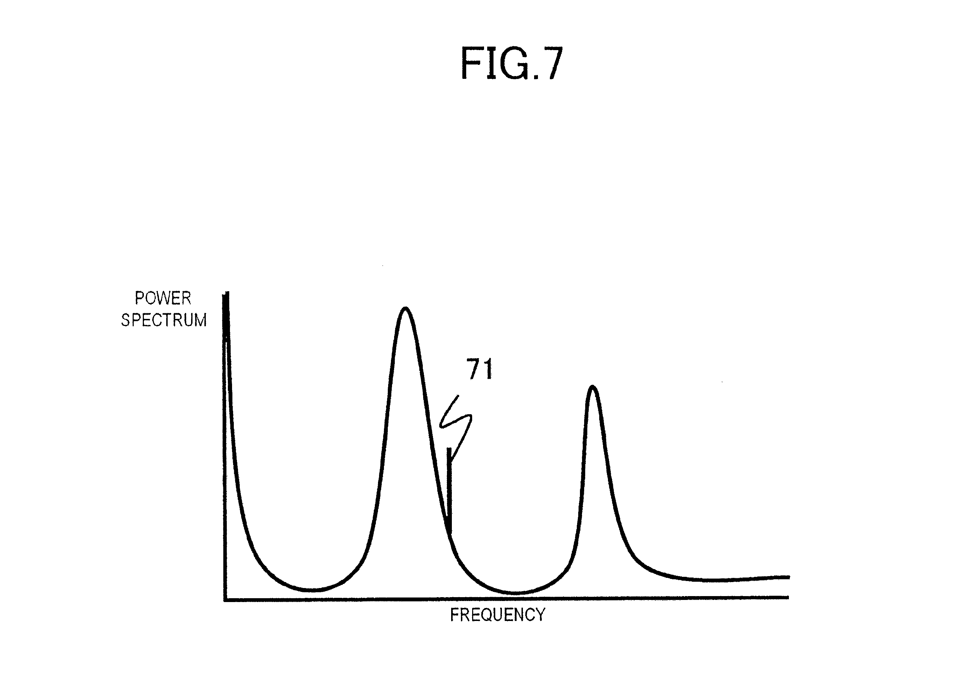

When the user determines that the estimated time phase used by the volume data construction processing unit 93 is not appropriate on the basis of the image displayed on the display unit 7 or the reliability, the correction value is set for the time phase (step 308). The user can input the correction value via the input unit 8. A GUI which is necessary for the input may also be displayed on the display unit 7. For example, a box, etc. for inputting the numeric value of the frequency or heart rate may be displayed on the screen, so that the numeric values can be input in the box. Or, as shown in Fig. 7, a cursor or mark 71 for selecting the frequency (or heart rate) may be displayed with the frequency spectrum, so that the user can arbitrarily move the cursor position. The frequency or heart rate which is selected by the user may also be displayed with a predetermined mark on the frequency spectrum.

As described above, when information such as the frequency or heart rate is input, the time phase correction unit 92 calculates all time phases of the cardiac movement of the fetus on the basis of the input frequency or heart rate, and transfers the calculated information to the volume data construction processing unit 93 (step 309). The volume data construction processing unit 93 selects plural sets of 2-dimensionanl image data of the same time phase on the basis of the corrected time phase information, and constructs a 3-dimensional ultrasonic image (step 305). That is, the image processing unit creates a 3-dimensional ultrasonic image using the time phase information corrected by the time phase correction unit 92.

The step of generating images by a desired rendering method on the basis of the constructed 3-dimensional ultrasonic images and displaying the images on the display unit 7 is the same as the case of not performing correction (steps 306 and 307). The user can also see the displayed images and change the time phase again.

As described above, the ultrasonic diagnostic apparatus of the present embodiment comprises:

the ultrasonic probe 1 configured to transmit ultrasonic beams and receive the reflected echo signals to/from an examination object,
the signal processing unit 4 configured to perform signal processing on the reflected echo signals and generate echo data;
the time phase estimation unit 91 configured to estimate the time phase information on the periodic movement of the examination object included in the echo data;
the image processing unit configured to create 3-dimensional ultrasonic images of the examination object for each time phase using the time phase information estimated by the time phase estimation unit 91 and the echo data; and
the display unit 7 configured to display the 3-dimensional ultrasonic images,
further comprising the time phase correction unit 92 configured to correct the time phase information estimated by the time phase estimation unit 91,
wherein the image processing unit creates 3-dimensional ultrasonic images using the time phase information corrected by the time phase correction unit. Also, the time phase correction unit 92 comprises the input unit for inputting arbitrary correction values, and sets the corrected value which is input to the input unit as a new time phase. The information related to the frequency displayed by the display unit 7 includes a graph in which the power spectrum is indicated by the longitudinal axis and the frequency or heart rate is indicated by the lateral axis.

Also, the image processing method for reproducing ultrasonic images using the 3-dimensional echo data corrected by an ultrasonic diagnostic apparatus includes:

a step of estimating the time phase (phase) of 3-dimensional echo data by calculating the periodicity included in the 3-dimensional echo data; and
a step of reproducing an ultrasonic image for each time phase by correcting the estimated time phase and extracting the data having the same time phase from the 3-dimensional echo data.

In other words, comprising a function for a user to arbitrarily correct the time phase estimated by the apparatus enables construction of time phase images properly corresponding to the movement cycles of an imaging object, thus the ultrasonic images can be displayed that are effective for diagnosis without repeating the examination. Also, the user can easily determine whether or not the estimated time phase is accurate, by displaying the information related to the time phase estimated by the apparatus.

<Embodiment 2>

The present embodiment is characterized in comprising a function which automatically corrects the time phase estimated by an apparatus. The rest of construction and operation is the same as the above-described Embodiment 1, thus the difference will be mainly described below.

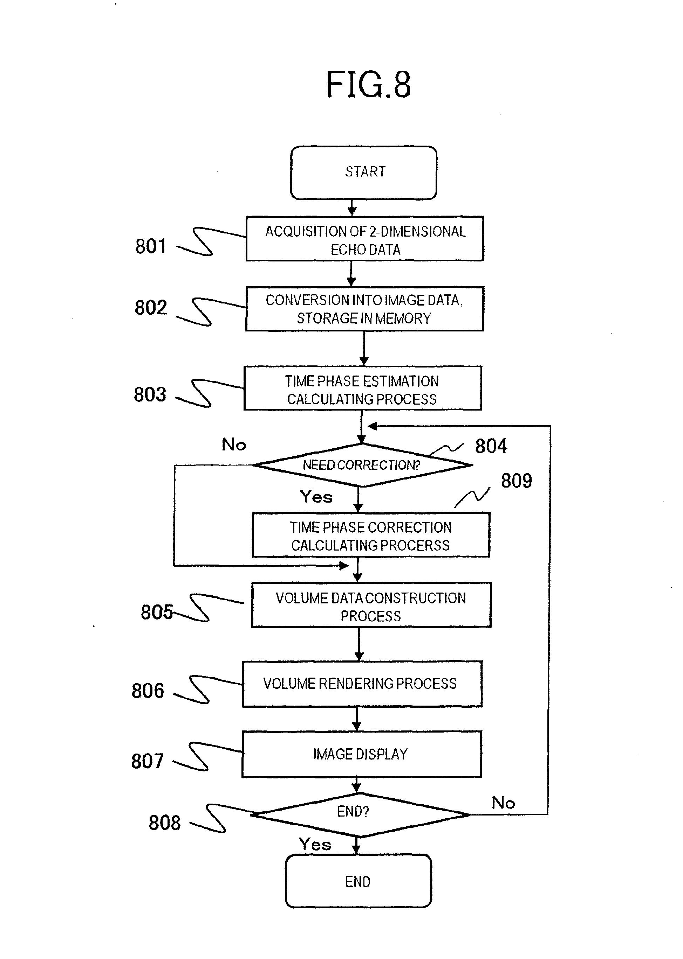

Fig. 8 shows the operation procedure of Embodiment 2. In the diagram, steps 801~803 and 805-807 correspond to steps 301~303 and 305-307 in which the similar processing is performed, thus the explanation thereof will be omitted and step 809 which the processing is different will be mainly described.

When the 3-dimensional images created by the volume rendering processing unit 94 are displayed on the display unit 7 in step 807 and the user determines that the images do not properly coincide with the movement cycles of the imaging object, he/she inputs a message saying "repeat imaging process (instruction for correction)" via the input unit 8 for indicating that the estimated time phase is not correct (steps 808 and 804). At this time, information such as the reliability may also be displayed with the image on the display unit 7 as in Embodiment 1. The command for correction is to be issued, for example by the GUI function provided in the display unit 7.

Here, the time phase correction unit 92 corrects the time phase information on the basis of the frequency information or heart rate information in the echo data. The image processing unit selects plural sets of 2-dimensional image data in the same time phase based on the time phase information corrected by the time phase correction unit 92, and constructs 3-dimensional ultrasonic images.

In concrete terms, since plural frequency peaks exist in the frequency spectrum acquired by the time phase estimation unit 91 in a majority of the cases that the time phase estimated by the time phase estimation unit 92 does not match the time phase of the imaging object, the time phase correction unit 92 sets the frequency having the second or third largest value of the power spectrum is set as a corrected frequency (step 809). Correcting all time phases in the cardiac movement of the imaged fetus using the corrected frequency is the same as Embodiment 1. While the frequency having a larger value of the power spectrum may be selected as the correction frequency when there are three or more candidate frequencies, the correction frequency may also be determined, by previously storing a range of the movement frequency (heart rate) of the imaging object or the upper limit or the lower limit in the memory 6, on the basis of the stored value. In this case, the frequency included in the range of frequencies stored in the memory 6 or the closest value thereto is to be selected as the correction frequency. In other words, the time phase correction unit 92 corrects the time phase information based on the frequency spectrum of echo data. The time phase correction unit 92 analyzes the echo data, and corrects the time phase information by setting the frequency having the second or third largest value in the power spectrum as the corrected time phase.

In the present embodiment also, it is preferable to display on the display unit 7 the selected correction frequency (heart rate) as a numeric value or a mark, etc. on the frequency spectrum.

While the case has been described above that the user determines the necessity of correction on the basis of the displayed image (step 804), the necessity of correction can also be automatically determined by the time phase correction unit 92 on the basis of the frequency spectrum acquired by the time phase estimation unit 91.

In concrete terms, when there is a single peak in the frequency spectrum calculated by the time phase estimation unit 91 using the Fourier transform, the estimation result of the time phase estimation unit 91 is set as "correct" and determined as "correction unnecessary". On the other hand, when there are plural frequency peaks, the range, the upper value or the lower value (set value) of the movement frequency (heart rate) of the imaging object previously stored in the memory 6 is compared with the estimated frequency, and determination is made as "correction necessary" if the estimated frequency is not within the set range.

When it is determined as "correction necessary", the message is displayed on the display unit 7. At this time, it is preferable that the frequency spectrum as shown in Fig. 7 is displayed, and the user can input the correction value on the basis of the displayed spectrum. The method to be used by the user for inputting the corrected value is the same as described in Embodiment 1.

The time phase correction unit 92 can also set the correction value automatically instead of manual input by the user. In this case, when it is determined that correction is necessary in step 804, the time phase correction unit 92 automatically sets the correction frequency and calculates the correction frequency, and all of the time phases are calculated on the basis of the set correction frequency in step 809 as described above. At this time, the time phase correction unit 92 performs the processing from the determination of correction necessity to the setting of correction values without any operation by the user. In this regard, however it may also be set so that the user can input correction necessity or correction values when the need arises.

The information related to the cycles to be displayed on the display unit 7 includes the evaluation of the time phase information estimated by the time phase estimation unit 91. The time phase correction unit 92 displays the information related to the post-correction cycles on top of the information related to the cycles acquired by the time phase estimation unit 91 displayed on the display unit 7.

<Embodiment 3>

The method for estimating time phases in the present embodiment is different from the one in Embodiment 1 and Embodiment 2. While the operation procedure in the present embodiment is the same as shown in Fig. 3, autocorrelation of the luminance value is to be calculated in step 303 for estimating the time phase from plural sets of 2-dimensional image data. Thus, the average value of the luminance values in the 2-dimensional image data is calculated as in Embodiment 1, and the waveform (Fig. 4(B)) which is the temporal variation of the average value is obtained. The autocorrelation function is calculated for the obtained waveform. Autocorrelation function $R(\tau)$ can be expressed using the following equation by setting the waveform of luminance variation as x(t), the delay of autocorrelation function as $\tau$, and the searching range as T.

$$R(\tau) = 1/T \int_{-T/2}^{T/2} x(t) x(t+\tau) dt$$

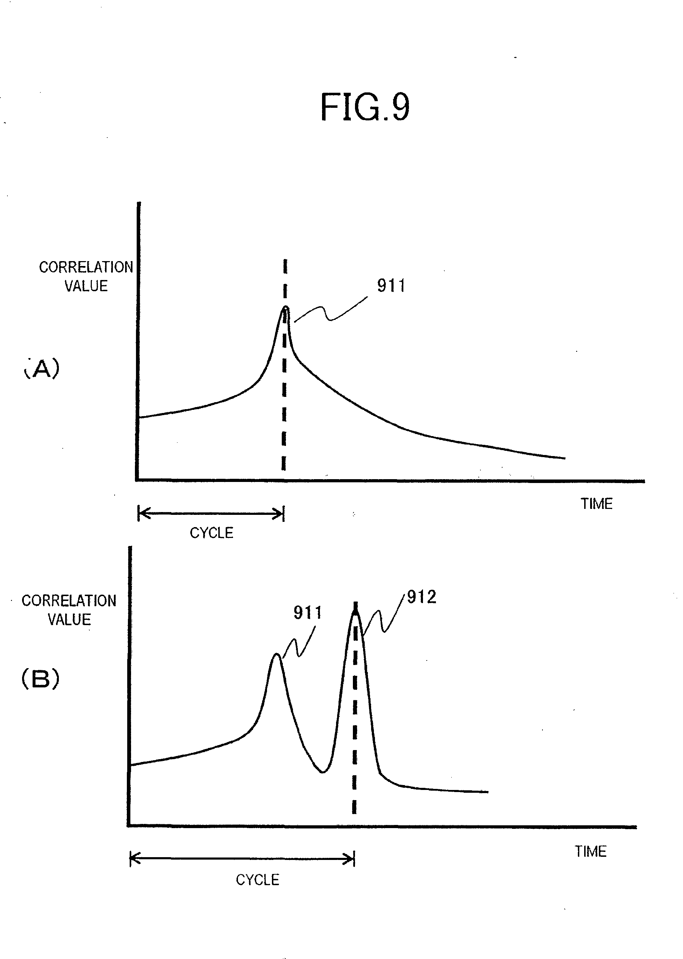

Autocorrelation function is a curve in which, for example the lateral axis indicates time and the longitudinal axis indicates the correlation value as shown in Figs. 9(A) and (B). In the case of the Fig. 9(A), the time from zero to a peak 911 on the lateral axis can be obtained as a cycle. Also as shown in Fig. 9 (B), when there are plural peaks 911 and 912, the time up to the peak having the highest correlation value (for example, 912) is obtained as a cycle.

In the present embodiment, as in the previously described embodiments, the time phase estimation 91 estimates all time phases of the heart of a fetus based on such obtained cycles, the volume data construction processing unit 93 creates the 3-dimensional image data for each time phase using the estimated time phase, then creates and displays rendering images based on the 3-dimensional image data. In the same manner, the reliability of the estimated time phase or the autocorrelation function (graph) may also be displayed with the displayed image.

On the basis of the information on the displayed image and/or the reliability, the time phase correction unit 92 corrects the time phase, and a 3-dimensional image is created again.

In accordance with the present embodiment, the time phase estimation unit 91 analyzes the time variation of the luminance in echo data, acquires the cycles included in the echo data, and estimates the time phase using a first cycle having the largest power spectrum or autocorrelation value, and the time phase correction unit 92 makes the information related to the cycle acquired by the time phase estimation unit 91 to be displayed on the display unit 7.

While the embodiments of the ultrasonic diagnostic apparatus related to the present invention has been described, the function of the control unit 9 in the ultrasonic diagnostic apparatus can also be comprised as the function of an image processing device which is separate from the ultrasonic diagnostic apparatus. In this case, raw data acquired by the ultrasonic diagnostic apparatus or image data converted by a conversion unit can be transferred to the image processing device via a wireless or wired communication unit or transportable medium, and processing such as time phase estimation, time phase correction, 3-dimensional image data creation and rendering can be performed by the image processing device.

Description of Reference Numerals

1 ultrasonic probe
2 transmission/reception switching unit
3 beam forming unit
4 signal processing unit
5 conversion unit
6 memory
7 display unit
8 input unit
9 control unit
91 time phase estimation unit
92 time phase correction unit
93 volume data construction processing unit
94 volume rendering processing unit

**Claims**

1. An ultrasonic diagnostic apparatus comprising:

   an ultrasonic probe configured to transmit ultrasonic beams and receives the reflected echo signals from an examination object;
   a signal processing unit configured to perform signal processing on the reflected echo signals and generate echo data;
   a time phase estimation unit configured to estimate time phase information on the periodic movement of the examination object included in the echo data;
   an image processing unit configured to create a 3-dimensional ultrasonic image of the examination object for each time phase using the time phase information estimated by the time phase estimation unit and the echo data; and
   a display unit configured to display the 3-dimensional ultrasonic image,
   further comprising a time phase correction unit configured to correct the time phase information estimated by the time phase estimation unit,
   wherein the image processing unit creates the 3-dimensional ultrasonic image using the time phase information corrected by the time phase correction unit.

2. The ultrasonic diagnostic apparatus according to claim 1, wherein the time phase correction unit comprises an input unit for inputting an arbitrary correction value, and sets the correction value inputted to the input unit as new time phase information.

3. The ultrasonic diagnostic apparatus according to claim 1, wherein:

   the time phase estimation unit analyzes the time variation of the luminance in echo data, acquires the cycles included in the echo data, and estimates the time phase using a first cycle having the largest power spectrum or autocorrelation value; and
   the display unit displays the information related to the cycles acquired by the time phase estimation unit.

4. The ultrasonic diagnostic apparatus according to claim 3, **characterized in that** the information related to the cycles displayed by the display unit includes a graph in which the longitudinal axis indicates the power spectrum and the lateral axis indicates the frequency or heart rate.

5. The ultrasonic diagnostic apparatus according to claim 3, **characterized in that** the information related to the cycles displayed by the display unit includes the evaluation of the time phase information estimated by the time phase estimation unit.

6. The ultrasonic diagnostic apparatus according to claim 3, wherein the time phase correction unit makes the information related to the post-correction cycles to be displayed on top of the information related to the cycles acquired by the time phase estimation unit displayed on the display unit.

7. The ultrasonic diagnostic apparatus according to claim 1, wherein:

   the time phase estimation unit analyzes the time variation of the luminance in the echo data, acquires one or more cycles included in the echo data, and estimates the time phase using a first cycle having the largest power spectrum or autocorrelation value; and
   the time phase correction unit, when there are plural cycles acquired by the time phase estimation unit and a second cycle which is most approximate to a previously set predictive value from among the plural cycles is different from the first cycle, corrects the time phase using the second cycle.

8. The ultrasonic diagnostic apparatus according to claim 1, wherein the image processing unit selects plural sets of 2-dimensional image data at the same time phase based on the time phase information corrected by the time phase correction unit, and constructs a 3-dimensinal ultrasonic image.

9. The ultrasonic diagnostic apparatus according to claim 1, wherein the time phase correction unit corrects time phase information based on the frequency information or heart rate information in the echo data.

10. The ultrasonic diagnostic apparatus according to claim 1, wherein the time phase correction unit analyzes the echo data, and corrects time phase information by setting the frequency having the second or third largest value of the power spectrum as a correction frequency.

11. An image processing method that reproduces ultrasonic images using the 3-dimensional echo data collected by an ultrasonic diagnostic apparatus, including:

a step of acquiring periodicity included in 3-dimensional echo data and estimating time phase (phase) of the 3-dimensinoal echo data; and
a step of correcting estimated time phase, extracting the data sets having the same time phase from the 3-dimensional echo  data sets and reproducing an ultrasonic image for each time phase.

# FIG.1

EP 2 659 839 A1

# FIG.2

CONVERSION UNIT — 5

91 — TIME PHASE ESTIMATION UNIT

92 — TIME PHASE CORRECTION UNIT

93 — VOLUME DATA CONSTRUCTION PROCESSING UNIT

94 — VOLUME RENDERING PROCESSING UNIT

CONTROL UNIT

9

MEMROY — 6

EP 2 659 839 A1

# FIG.3

```
                    ┌─────────────┐
                    │    START    │
                    └─────────────┘
                           │
                           ▼
        ┌──────────────────────────────────────┐
  301   │ ACQUISITION OF 2-DIMENSIONAL          │
        │ ECHO DATA                             │
        └──────────────────────────────────────┘
                           │
                           ▼
        ┌──────────────────────────────────────┐
  302   │ CONVERSION INTO IMAGE DATA,           │
        │ STORAGE IN MEMORY                     │
        └──────────────────────────────────────┘
                           │
                           ▼
        ┌──────────────────────────────────────┐
  303   │ TIME PHASE ESTIMATION                 │
        │ CALCULATING PROCESS                   │
        └──────────────────────────────────────┘
                           │
      No                   ▼              304
        ◄──────────◇ NEED CORRECTION? ◇
                    │                    309
                Yes ▼
        ┌──────────────────────────────────────┐
        │ TIME PHASE CORRECTION                 │
        │ CALCULATING PROCESS                   │
        └──────────────────────────────────────┘
                           │
        ┌──────────────────────────────────────┐
  305   │ VOLUME DATA CONSTRUCTION              │
        │ PROCESS                               │
        └──────────────────────────────────────┘
                           │
                           ▼
        ┌──────────────────────────────────────┐
  306   │ VOLUME RENDERING PROCESS              │
        └──────────────────────────────────────┘
                           │
                           ▼
        ┌──────────────────────────────────────┐
  307   │ IMAGE DISPLAY                         │
        └──────────────────────────────────────┘
                           │
                           ▼                    No
  308         ◇ SET CORRECTION? ◇ ─────────►
                           │
                       Yes ▼
                    ┌─────────────┐
                    │     END     │
                    └─────────────┘
```

# FIG.4

(A)

400

E

TIME

LUMINANCE
VALUE

(B)

TIME

POWER
SPECTORUM

(C)

411

FREQUENCY

POWER
SPECTORUM

(D)

411

412

FREQUENCY

EP 2 659 839 A1

# FIG.5

(A)

TIME
PHASE

CYCLE

TIME

(B)

500

TIME

(C)

IMAGE OF
TIME
PHASE 1

IMAGE OF
TIME
PHASE 2

IMAGE OF
TIME
PHASE 3

IMAGE OF
TIME
PHASE 4

15

# FIG.6

POWER SPECTORUM

FREQUENCY (HEART RATE)

DISPLAY SCREEN

VOLUME DATA DISPLAY

RELIABILITY: HIGH

(A)

POWER SPECTORUM

FREQUENCY (HEART RATE)

VOLUME DATA DISPLAY

RELIABILITY: MIDDLE

(B)

POWER SPECTORUM

FREQUENCY (HEART RATE)

VOLUME DATA DISPLAY

RELIABILITY: LOW

(C)

EP 2 659 839 A1

# FIG.7

# FIG.8

START

801    ACQUISITION OF 2-DIMENSIONAL ECHO DATA

802    CONVERSION INTO IMAGE DATA, STORAGE IN MEMORY

803    TIME PHASE ESTIMATION CALCULATING PROCESS

804    NEED CORRECTION?

No

Yes

809    TIME PHASE CORRECTION CALCULATING PROCERSS

805    VOLUME DATA CONSTRUCTION PROCESS

806    VOLUME RENDERING PROCESS

807    IMAGE DISPLAY

808    END?

No

Yes

END

# FIG.9

(A)

CORRELATION VALUE

911

CYCLE

TIME

(B)

CORRELATION VALUE

911

912

CYCLE

TIME

## INTERNATIONAL SEARCH REPORT

| International application No. |
|---|
| PCT/JP2011/078233 |

A. CLASSIFICATION OF SUBJECT MATTER
*A61B8/00*(2006.01)i

According to International Patent Classification (IPC) or to both national classification and IPC

B. FIELDS SEARCHED

Minimum documentation searched (classification system followed by classification symbols)
A61B8/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | JP 2010-193945 A (Toshiba Corp.), 09 September 2010 (09.09.2010), entire text; all drawings; particularly, paragraphs [0065] to [0071] (Family: none) | 1-11 |
| A | JP 2010-119587 A (Toshiba Corp.), 03 June 2010 (03.06.2010), entire text; all drawings (Family: none) | 1-11 |
| A | JP 2005-74225 A (GE Medical Systems Global Technology Co., L.L.C.), 24 March 2005 (24.03.2005), entire text; all drawings & US 2005/0049503 A1 & DE 102004040411 A & DE 202004021722 U | 1-11 |

☒ Further documents are listed in the continuation of Box C.   ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 21 December, 2011 (21.12.11) | 10 January, 2012 (10.01.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

| International application No. |
| --- |
| PCT/JP2011/078233 |

C (Continuation).   DOCUMENTS CONSIDERED TO BE RELEVANT

| Category* | Citation of document, with indication, where appropriate, of the relevant passages | Relevant to claim No. |
| --- | --- | --- |
| P,A | JP 2011-62404 A  (Aloka Co., Ltd.),<br>31 March 2011 (31.03.2011),<br>entire text; all drawings<br>& US 2010/0262006 A1    & EP 2241256 A2<br>& CN 101897601 A | 1-11 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005074225 A

**Non-patent literature cited in the description**

- *Capacitive Michromachined Ultrasonic Transducer: IEEE Trans. Ultrason. Ferroelect. Freq. Contr.,* May 1998, vol. 45, 678-690